# EUROPÄISCHE PATENTANMELDUNG

(11) **EP 4 727 001 A1**
(43) Veröffentlichungstag der Anmeldung: **15.04.2026**
(21) Anmeldenummer: 24205205.8
(22) Anmeldetag: 08.10.2024
(51) Int. Cl.: H02P 8/16, A61M 5/142, A61M 5/172, H02P 8/22, H02P 8/38

(54) **ANSTEUERUNGSVORRICHTUNG FÜR EINEN SCHRITTMOTOR EINER INFUSIONSPUMPE, INFUSIONSPUMPE, ANSTEUERUNGSVERFAHREN, COMPUTERPROGRAMM UND COMPUTERLESBARES SPEICHERMEDIUM**

(71) Anmelder: B. Braun Melsungen AG, 34212 Melsungen (DE)
(72) Erfinder: LUKAS, Frank, 34121 Kassel (DE)
(74) Vertreter: Winter, Brandl - Partnerschaft mbB

(57) **Zusammenfassung**

Steuerungsvorrichtung (10, 31) für einen Schrittmotor (30) einer Infusionspumpe (33), mit folgenden Vorrichtungs-Funktionsbereichen: einem Empfangsbereich (11), der dafür vorgesehen und ausgebildet ist, Sollpositionsdaten für die Ansteuerung des Schrittmotors zu empfangen; einem Bestimmungsbereich (12), der dafür vorgesehen und ausgebildet ist, Ansteuerungsdaten für den Schrittmotor auf Basis der Sollpositionsdaten zu bestimmen; einem Ansteuerungsbereich (13), der dafür vorgesehen und ausgebildet ist, den Schrittmotor auf Basis der bestimmten Ansteuerungsdaten anzusteuern; wobei der Bestimmungsbereich (12) vorgesehen und ausgebildet ist, die Ansteuerungsdaten derart zu bestimmen, sodass beim Betrieb der Infusionspumpe (33) eine Reduzierung der Geräuschbildung erzielt wird.

## Beschreibung

### Technisches Gebiet

Die vorliegende Erfindung betriff eine Ansteuerungsvorrichtung für einen Schrittmotor einer Infusionspumpe, ein Ansteuerungsverfahren, ein Computerprogramm sowie ein computerlesbares Speichermedium.

### Technischer Hintergrund

Infusionspumpen mit Schrittmotoren sind im Stand der Technik grundsätzlich bekannt. Der Schrittmotor treibt dabei die Pumpe an, sodass die Infusionsflüssigkeit gefördert wird. Die Infusionspumpen werden dabei in einer medizinischen Umgebung eingesetzt, in welcher es ruhig ist, sodass auch vermeintlich leise Geräusche als Störung für Patienten wahrnehmbar sind. Die Infusionspumpen werden im Rahmen einer Infusionstherapie verwendet. Insbesondere im nächtlichen Betrieb von Intensivstationen und Vorhandensein mehrerer Infusionspumpen an einem Bettplatz kann dies zu einer unangenehmen Situation für den Patienten führen und ihn beim Genesungsprozess negativ beeinträchtigen.

Es besteht daher ein Bedarf an einer besseren Ansteuerung von Schrittmotoren für Infusionspumpen.

### Kurzdarstellung der vorliegenden Offenbarung

Es ist daher die Aufgabe der vorliegenden Offenbarung, die Nachteile aus dem Stand der Technik zu verringern und insbesondere eine Ansteuerungsvorrichtung für einen Schrittmotor einer Infusionspumpe bereitzustellen, die einen möglichst leisen Betrieb der Infusionspumpe ermöglicht.

Die Aufgabe der vorliegenden Offenbarung wird durch eine Ansteuerungsvorrichtung mit den Merkmalen des Patentanspruchs 1 sowie den nebengeordneten Ansprüchen gelöst. Vorteilhafte Ausgestaltungen sind Gegenstand der Unteransprüche und/oder in der Beschreibung und den Figuren offenbart.

Gemäß der vorliegenden Offenbarung wird eine Ansteuerungsvorrichtung für einen Schrittmotor einer Infusionspumpe, mit folgenden Vorrichtungs-Funktionsbereichen bereitgestellt: einem Empfangsbereich, der dafür vorgesehen und ausgebildet ist, Sollpositionsdaten für die Ansteuerung des Schrittmotors zu empfangen; einem Bestimmungsbereich, der dafür vorgesehen und ausgebildet ist, Ansteuerungsdaten für den Schrittmotor auf Basis der Sollpositionsdaten zu bestimmen; einem Ansteuerungsbereich, der dafür vorgesehen und ausgebildet ist, den Schrittmotor auf Basis der bestimmten Ansteuerungsdaten anzusteuern; wobei die Ansteuerungsdaten derart bestimmt werden, sodass beim Betrieb der Infusionspumpe eine Reduzierung der Geräuschbildung erzielt wird.

Der Begriff Schrittmotor meint vorliegend vorzugsweise einen Hybridschrittmotor. Der Hybridschrittmotor umfasst vorzugsweise einen Rotor mit Permanentmagnet mit zwei oder drei gezahnten Weicheisenkränzen. Die Weicheisenkränze sind dabei vorzugsweise jeweils um einen halben Schritt versetzt. Der Hybridschrittmotor kann 2-, 3- oder mehr-phasig sein. Vorzugsweise ist der Schrittmotor 2-phasig. Der Hybridschrittmotor umfasst einen Stator. Der Stator umfasst vorzugsweise 8 Magnetpole beim 2-phasigen Hybridschrittmotor und beispielsweise 10 Magnetpole beim 5-phasigen Hybridschrittmotor. Die Magnetpole können Zähne aufweisen. Der Rotor bzw. der gezahnte Weicheneisenkranz weist dabei eine Anzahl n an Zähnen auf. Die Anzahl n kann eine aus den folgenden sein: 50, 100, 200. Die Schrittweite eines Vollschritts hängt von der Anzahl der Zähne ab. Die Schrittweite entspricht dabei beispielsweise bei 50 Zähnen 7,2°, 100 Zähnen 3,6°, bei 200 Zähnen 1,8°. Schrittmotoren bewegen sich in diskreten Schritten, die durch elektrische Impulse, sogenannte Steuerimpulse, gesteuert werden.

Der Begriff Infusionspumpe meint vorliegend insbesondere eine Pumpe, die eingerichtet ist, eine Flüssigkeit zur medizinischen Behandlung für eine Infusion bereitzustellen. Die Infusionspumpe kann dabei eine Kolbenpumpe sein. Die Kolbenpumpe kann eine Spritzenpumpe umfassen, wobei die Spritzenpumpe einen Kolben und einen Zylinder umfassen kann. Die Infusionspumpe wird dabei über den Schrittmotor angetrieben. Zwischen Schrittmotor und Infusionspumpe kann ein Getriebe zwischengeschaltet werden, sodass die Rotationsbewegung in eine Linearbewegung übersetzt wird. Die Infusionspumpe kann dabei einer Spritze ähneln. Der Kolben kann mit einem Drucksensor, beispielsweise einem Dehnmessstreifen versehen sein. Dadurch kann der anliegende Pumpendruck innerhalb der Infusionspumpe bestimmt werden.

Der Begriff Sollpositionsdaten meint vorliegend insbesondere eine Position, die der Kolben innerhalb der Infusionspumpe einnehmen soll. Die Sollpositionsdaten können weiterhin zeitliche Angaben aufweisen, innerhalb der der Kolben diese Position innehaben soll. Die Sollpositionsdaten können ein gewünschtes Verfahrprofil für den Kolben beinhalten. Die Sollpositionsdaten können in Form eines Programms vorliegen. Das Programm kann zunächst in Form einer gewünschten Förderrate oder eines Förderratenverlaufs vorgegeben werden. Die Ansteuerungsvorrichtung kann aus diesem Programm dann vorzugsweise über Kenntnis der Kolbengeometrie sowie der Verfahrstrecke des Kolbens pro Vollschritt bzw. Mikroschritt eine gewünschte Anzahl an Schritten pro Zeiteinheit ableiten.

Der Begriff Empfangsbereich meint vorliegend insbesondere eine Schnittstelle. Die Schnittstelle kann hardware- und/oder software-basiert sein.

Der Begriff Bestimmungsbereich meint vorliegend einen Funktionsbereich, der aus den Sollpositionsdaten Ansteuerungsdaten ableitet. Hierzu kann der Funktionsbereich eine Logik aufweisen, sodass er Ansteuerungsdaten erzeugen kann.

Der Begriff Ansteuerungsdaten meint vorliegend die Befehle zur Steuerung einer Leistungselektronik. Die Befehle können insbesondere die Vorgabe einer Schrittgeschwindigkeit bzw. Schrittfrequenz, einer Schrittanzahl, einer Beschleunigung, eines maximalen Stromwertes, eines Stromwertes zum Drehen des Schrittmotors, eines Haltestroms zur Sicherstellung der Position im Ruhestand, eines Anlaufstroms, einer Pulsweite bzw. Pulsbreite, einer Spannungswertes bzw. Stromwertes, einen Schrittmodus (Vollschritt, Teilschritt, Mikroschritt) umfassen. Die Leistungselektronik schaltet dann entsprechend die Magnetpole bzw. die zugehörigen Phasenwicklungen und beaufschlagt sie mit Strom. Mit anderen Worten ausgedrückt wird eine entsprechende Spannung an die jeweilige Phasenwicklung angelegt, so dass der gewünschte Strom fließt.

Der Begriff Ansteuerungsbereich meint vorliegend eine Schaltung, die eine Leistungselektronik eines Schrittmotors ansteuert.

Der Erfindung liegt die Erkenntnis zugrunde, dass der Schrittmotor durch seinen schrittweisen Betrieb zu einer Anregung der strukturellen Komponenten der Infusionspumpe, beispielsweise Getriebe, Gehäuse, Kolben oder Zylinder, führen kann. Hierbei wirkt jeder Schritt letztlich wie ein Kraftimpuls auf die Infusionspumpe. Die Kraft führt letztlich zu einer mechanischen Schwingung in den Strukturkomponenten, die sich an der Oberfläche der strukturellen Komponenten in Luftschall ausbreitet und somit für ein menschliches Gehör in der Umgebung wahrnehmbar ist. Diese Geräusche sind als störend, wenn nicht sogar heilungsverzögernd für Patienten in einer Intensivstation einzustufen. Die erfindungsgemäße Ansteuerungsvorrichtung löst dieses Problem, indem sie die Parameter für die Ansteuerung des Schrittmotors derart wählt, dass die Anregung der strukturellen Komponenten reduziert wird und so auch die Geräuschentwicklung. Dies wirkt sich vorteilhaft auf die Geräuschentwicklung und den Betrieb der Infusionspumpe aus.

Gemäß einer bevorzugten Ausführungsform können die Ansteuerungsdaten derart bestimmt werden, dass eine äquidistante Pulsweite vorliegt.

Der Begriff Pulsweite bei einem Schrittmotor meint eine Dauer, für die ein Steuerimpuls an den Motor gesendet wird. Die Pulsweite beeinflusst, wie lange der Motorstrom in einer bestimmten Richtung fließt, und damit die Position und Geschwindigkeit des Schrittmotors. Eine längere Pulsweite führt dazu, dass der Motor mehr Energie erhält und den Schritt vollständiger und mit mehr Drehmoment ausführt. Umgekehrt führt eine kürzere Pulsweite zu einer schnelleren, aber möglicherweise weniger kraftvollen Bewegung.

Eine äquidistante Pulsweite meint insbesondere, dass sowohl die Dauer der gesendeten Stromimpulse (d.h. Pulse) als auch der zeitliche Abstand der Stromimpulse gleich groß sein können. Mit anderen Worten wird der Schrittmotor mit einer konstanten Geschwindigkeit betrieben.

Auf diese Weise können vorteilhafterweise arrhythmische Anregungen der Infusionspumpe vermieden werden.

Gemäß einer bevorzugten Ausführungsform können die Ansteuerungsdaten derart bestimmt werden, dass eine Auflösung für einen Vollschritt in Abhängigkeit einer Förderrate der Infusionspumpe angepasst wird.

Der Begriff Auflösung für einen Vollschritt meint vorliegend insbesondere die weitere Unterteilung eines Vollschritts in Mikroschritte im Mikroschrittbetrieb. Der Mikroschritt bei einem Schrittmotor bezeichnet eine Technik, bei der die normalen Schrittpositionen des Motors in kleinere Zwischenschritte unterteilt werden. Dadurch kann der Motor sich feiner und sanfter bewegen, was die Auflösung und Genauigkeit der Positionierung erhöht. In einem herkömmlichen Schrittmotor entspricht jeder Schritt einer festen Winkelposition. Bei einem Mikroschrittbetrieb wird dieser Winkel weiter aufgeteilt, indem die Phasenströme in den Motorwicklungen so gesteuert werden, dass sie Zwischenpositionen erreichen. Die feinere Auflösung wird im Mikroschrittbetrieb durch die Vorgabe eines stufen- und sinusförmigen Steuerimpulses erzielt. Zum Beispiel kann ein Schrittmotor, der normalerweise 200 Schritte pro Umdrehung macht (entspricht 1,8° pro Schritt), im Mikroschrittbetrieb auf 1/10 Mikroschritte eingestellt werden und so 2000 Schritte pro Umdrehung ausführen.

Die Förderrate der Pumpe meint vorliegend ein Volumen pro Zeiteinheit. Bei einer großen Förderrate bewegt der Schrittmotor den Kolben vorzugsweise schneller in eine gewünschte Position, sodass mehr Volumen pro Zeiteinheit gefördert wird und umgekehrt.

Durch die höhere Auflösung der Mikroschritte wird eine verbesserte Laufruhe erzielt, da der Schrittmotor sanft an die nächste Motorposition herangeführt wird. Bei höheren Förderraten kann eine interne Timerauflösung zu gering sein, um alle Mikroschritte auszugeben. In diesem Fall wird dynamisch auf kleinere Auflösungen "heruntergeschaltet".

Gemäß einer bevorzugten Ausführungsform können die Ansteuerungsdaten derart bestimmt werden, dass ein Maximalwert für einen Motorstrom des Schrittmotors vorgegeben wird.

Die Begrenzung des Motorstroms führt entsprechend zu einer Begrenzung des Motordrehmoments. Hierdurch wird mit anderen Worten auch die Kraft der Anregung auf die Struktur begrenzt, was zu geringen Anregungsimpulsen führt. Dies bewirkt dann wiederum eine geringere Geräuschentwicklung. Mit anderen Worten, es erfolgt kein hartes Anschlagen an die elektronische Rastposition des Schrittes.

Gemäß einer bevorzugten Ausführungsform können die Ansteuerungsdaten derart bestimmt werden, dass ein Motorstrom in Abhängigkeit einer Schrittgeschwindigkeit des Schrittmotors angepasst wird.

Bei Vorliegen einer Begrenzung des Motorstroms auf ein Minimum wird zwar die Geräuschentwicklung reduziert, es besteht jedoch gleichzeitig die Gefahr, dass ein Drehmomentabriss bei Laständerungen auftreten kann. Mit anderen Worten ausgedrückt, der Schrittmotor bewegt sich nicht weiter. Das kann beispielsweise der Fall sein, wenn die Infusion ausgegeben wird (z.B. Änderung des Systemdrucks zu Beginn der Bolusgabe (i.e. Infusionsmittel) oder Widerstand im Körper). Dabei wird die im Regelfall auch die Schrittgeschwindigkeit entsprechend angepasst. Für einen derartigen Fall kann es vorteilhaft sein, den Motorstrom, insbesondere die Maximalwerte für den Motorstrom, dynamisch anzupassen, um einen Drehmomentabriss zu verhindern und trotzdem bei reduzierter Geräuschentwicklung die Infusionspumpe betriebssicher zu betreiben.

Gemäß einer bevorzugten Ausführungsform können die Ansteuerungsdaten derart bestimmt werden, dass der Motorstrom in Abhängigkeit von einem anliegenden Druck innerhalb der Infusionspumpe dynamisch angepasst wird.

Bei Vorliegen einer Begrenzung des Motorstroms auf ein Minimum wird zwar die Geräuschentwicklung reduziert, es besteht jedoch gleichzeitig die Gefahr, dass ein Drehmomentabriss bei Laständerungen auftreten kann. Mit anderen Worten ausgedrückt, der Schrittmotor bewegt sich nicht weiter. Das kann beispielsweise der Fall sein, wenn der Systemdruck in der Infusionspumpe so hoch ist, dass die vom Schrittmotor aufgebrauchte Drehmoment nicht ausreicht, Kolben weiter zu verfahren. Vorliegend wird deswegen der anliegende Druck innerhalb der Pumpe gemessen, beispielsweise über einen Drucksensor. Der gemessene Druckwert kann zur dynamischen Anpassung des Motorstroms, insbesondere des Maximalwertes für den Motorstrom herangezogen werden. Bei hohem Druck wird entsprechend der Motorstrom bzw. Maximalwert des Motorstroms dynamisch nach oben abgeändert. Dadurch kann ein Drehmomentabriss verhindert werden und trotzdem bei reduzierter Geräuschentwicklung die Infusionspumpe betriebssicher betrieben werden.

Gemäß einer bevorzugten Ausführungsform können die Ansteuerungsdaten eine Vorgabe eines Haltestroms umfassen, der während einer Schrittpause auf den Schrittmotor wirken soll, sodass eine elektronische Rastposition während der Schrittpause aufrechterhalten wird.

Der Begriff Schrittpause meint vorliegend die Zeit zwischen einem vollendeten Schritt, insbesondere Mikroschritt, und dem nächsten Schritt, insbesondere Mikroschritt. Die elektronische Rastposition ist nicht identisch mit der magnetischen Rastposition. Dadurch kann ein geringfügiges Zurückfallen bzw. Vorauseilen der Motorposition während der Schrittpausen auftreten, was erheblich zur Geräuschbildung beitragen kann. Der Haltestrom sorgt dafür, dass die elektronische Rastposition während der Schrittpause nicht aufgegeben wird. Dies wirkt sich vorteilhaft auf die Geräuschposition aus.

Gemäß einer bevorzugten Ausführungsform können die Ansteuerungsdaten derart bestimmt werden, dass einzelne Mikroschritte eines Vollschritts kontinuierlich ausgegeben werden.

Durch kontinuierliche Ausgabe an Mikroschritten werden Unterbrechungen der Bewegung verhindert. Die kontinuierliche Schrittausgabe kann eine gleichmäßige Drehung und dadurch eine konstante Geschwindigkeit bewirken. Dies kann sich positiv auf die Geräuschentwicklung bei höheren Schrittfrequenzen auswirken. Insbesondere in Kombination mit einer Motorstrombegrenzung kann die Gefahr einer thermischen Belastung ausgeschlossen werden. Vorzugsweise wird die kontinuierliche Schrittausgabe im Netzbetrieb durchgeführt. Alternativ kann die kontinuierliche Schrittausgabe im Batteriebetrieb durchgeführt werden.

Gemäß einer bevorzugten Ausführungsform können die Ansteuerungsdaten derart bestimmt werden, dass bei Auftreten eines Drehmomentabrisses ein Maximalwert für den Motorstrom für vorbestimmte Anzahl an Schritten erhöht wird und anschließend wieder erniedrigt wird, sodass ein erneuter Drehmomentabriss vermieden und eine weitere Geräuschentwicklung reduziert wird.

Im Fall eines verloren gegangenen Schrittes kann es bevorzugt sein, diesen nachzuholen. Ein Schrittmotor kann hierzu eine Lichtschranke aufweisen, die jeden Schritt zählt. Bei einem Abriss eines Drehmoments kann der Ansteuerungsbereich eingerichtet sein, den Schrittmotor um eine vorbestimmte Anzahl an Schritten, vorzugsweise vier Schritte auf das nächste passende Phasensignal zurück anzusteuern. Die Lichtschranke erkennt dabei beispielsweise drei Schritte und einen verlorengegangenen Schritt. Nach Erkennung verlorener Schritte werden diese erneut in Auftrag gegeben, sogenannte Rekuperation. In einem solchen Fall kann es vorteilhaft sein, den Maximalwert für den Motorstrom für eine bestimmte Anzahl an Folgeschritten anzuheben und anschließend wieder abzusenken, sodass ein mögliches Hindernis, das für den Drehmomentabriss verantwortlich war, überwunden wird. Im Anschluss wird dann wieder bei niedrigeren Maximalwert für den Motorstrom ein leiser Betrieb gewährleistet. Dies wirkt sich insgesamt positiv auf die Betriebssicherheit und Geräuschentwicklung der Infusionspumpe aus. Der Drehmomentabriss kann beispielsweise durch Slipstick-Effekte beim Getriebe oder des Schrittmotors auftreten oder generell bei Schwergängigkeit der beweglichen Teile innerhalb des Antriebsstrangs. Insgesamt kann die Ausführungsform ermöglichen, dass ein leiser Betrieb durch geringen Motorstrom ermöglicht und im Falle eines unvorhersehbaren Drehmomentabrisses, der Motorstrom kurzzeitig erhöht wird, sodass ein zwar kurzzeitig lauter aber insgesamt sicherer Betrieb gewährleistet wird.

Gemäß einem weiteren Aspekt betrifft die vorliegende Offenbarung ein Ansteuerungsverfahren zur Ansteuerung eines Schrittmotors einer Infusionspumpe, umfassend: Empfangen von Sollpositionsdaten für die Ansteuerung des Schrittmotors; Bestimmen von Ansteuerungsdaten für den Schrittmotor auf Basis der Sollpositionsdaten; Ansteuern des Schrittmotors auf Basis der bestimmten Ansteuerungsdaten; wobei die Ansteuerungsdaten derart bestimmt werden, sodass beim Betrieb der Infusionspumpe eine Reduzierung der Geräuschbildung erzielt wird. Das Ansteuerungsverfahren kann auch als ein computer-implementiertes Verfahren bezeichnet werden. Das Ansteuerungsverfahren kann entsprechend den Aspekten der Ansteuerungsvorrichtung weitergebildet werden.

Gemäß einem weiteren Aspekt betrifft die vorliegende Offenbarung ein Computerprogramm, das Anweisungen aufweist, die bei Ausführung durch einen Computer diesen veranlassen, das oben beschriebene Ansteuerungsverfahren auszuführen.

Gemäß einem weiteren Aspekt betrifft die vorliegende Offenbarung ein computerlesbares Speichermedium, in dem das oben beschriebene Computerprogramm gespeichert ist.

Funktionsbereiche einer Vorrichtung und/oder von Vorrichtungen gemäß einem oder mehrerer Aspekte können unter Verwendung von Hardware, Software und/oder einer Kombination davon implementiert werden. Die Funktionsbereiche einer Vorrichtung und/oder von Vorrichtungen können einteilig oder mehrteilig sein. Hardware-Vorrichtungen können beispielsweise durch Verarbeitungsschaltungen wie einen Prozessor, eine Zentraleinheit (CPU), einen Controller, eine arithmetische Logikeinheit (ALU), einen digitalen Signalprozessor, einen Mikrocomputer, ein feldprogrammierbares Gate-Array (FPGA), ein System-on-Chip (SoC), eine programmierbare Logikeinheit, einen Mikroprozessor oder jede andere Vorrichtung, die in der Lage ist, auf Befehle zu reagieren und diese in einer festgelegten Weise auszuführen, implementiert werden.

Die Funktionsbereiche einer Vorrichtung oder von Vorrichtungen können eine oder mehrere Schnittstellenschaltungen umfassen. In einigen Beispielen können die Schnittstellenschaltungen verdrahtete oder drahtlose Schnittstellen umfassen, die mit einem lokalen Netz (LAN), dem Internet, einem Weitverkehrsnetz (WAN) oder Kombinationen davon verbunden sind. Die Funktionalität eines bestimmten Geräts oder einer Einheit der vorliegenden Offenbarung kann auf mehrere Einheiten oder Geräte verteilt werden, die über Schnittstellenschaltungen verbunden sind.

Die Funktionsbereiche einer Vorrichtung oder von Vorrichtungen gemäß einem oder mehreren Aspekten können auch ein oder mehrere Speichergeräte umfassen. Bei der einen oder den mehreren Speichervorrichtungen kann es sich um materielle oder nichttransitorische computerlesbare Speichermedien handeln, wie Direktzugriffsspeicher (RAM), Festwertspeicher (ROM), ein permanentes Massenspeichergerät (z. B. ein Festplattenlaufwerk), ein Solid-State-Gerät (z. B. NAND flash) und/oder jeder andere Datenspeichermechanismus, der Daten speichern und aufzeichnen kann. Die eine oder mehrere Speichervorrichtungen können zum Speichern von Computerprogramme, Programmcode, Anweisungen oder eine Kombination davon eingerichtet sein.

Die hier beschriebenen Erläuterungen und Vorteile einzelner Aspekte gelten sinngemäß auch für die anderen Aspekte. So sind beispielsweise die Erläuterungen und Vorteile der Ansteuerungsvorrichtung für einen Schrittmotor einer Infusionspumpe auch relevant für das Ansteuerungsverfahren zur Ansteuerung eines Schrittmotors einer Infusionspumpe. Verschiedene beispielhafte Merkmale der Ausführungsformen können erfindungsgemäß kombiniert werden, wo immer dies technisch sinnvoll und machbar ist.

### Kurzbeschreibung der Figuren

Die vorliegende Offenbarung wird nachfolgend mit Hilfe von Figuren erläutert. Es zeigen.
Figur 1 eine schematische Darstellung der Ansteuerungsvorrichtung, und
Figur 2 eine schematische Darstellung einer Infusionspumpe mit einem Schrittmotor und einer Infusionspumpe.

### Detaillierte Beschreibung von bevorzugten Ausführungsformen

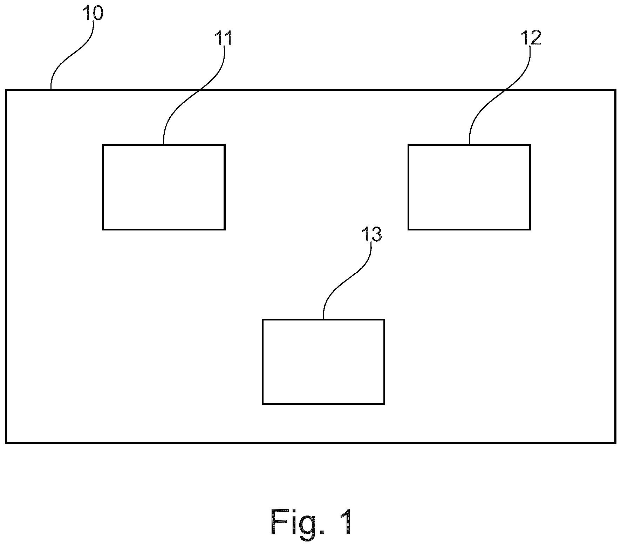

Figur 1 zeigt eine schematische Darstellung der Ansteuerungsvorrichtung 10.

Die Ansteuerungsvorrichtung 10 umfasst vorliegend eine Vielzahl an Vorrichtungs-Funktionsbereichen. Die Ansteuerungsvorrichtung 10 ist als Steuerung ausgebildet. Die Ansteuerungsvorrichtung 10 ist vorliegend ein Mikroprozessor, der mit Schnittstellen zum Datenempfang und zur Datenausgabe ausgestattet ist. Die Ansteuerungsvorrichtung 10 umfasst einen Empfangsbereich 11, der dafür vorgesehen und ausgebildet ist, Sollpositionsdaten für die Ansteuerung des Schrittmotors (in Fig. 1 nicht gezeigt) zu empfangen. Der Empfangsbereich 11 ist vorliegend als Schnittstelle zum Datenaustausch ausgeführt. Die Ansteuerungsvorrichtung 10 umfasst vorliegend einen Bestimmungsbereich 12, der dafür vorgesehen und ausgebildet ist, Ansteuerungsdaten für den Schrittmotor auf Basis der Sollpositionsdaten zu bestimmen. Die Ansteuerungsvorrichtung 10 umfasst vorliegend einen Ansteuerungsbereich 13, der dafür vorgesehen und ausgebildet ist, den Schrittmotor auf Basis der bestimmten Ansteuerungsdaten anzusteuern. Die Ansteuerungsdaten werden durch den Bestimmungsbereich 12 dabei derart bestimmt, sodass beim Betrieb der Infusionspumpe (in Fig. 1 nicht gezeigt) eine Reduzierung der Geräuschbildung erzielt wird.

Die Ansteuerungsdaten können dazu beispielsweise derart bestimmt werden, dass eine äquidistante Pulsweite vorliegt.

Außerdem können die Ansteuerungsdaten derart bestimmt werden, dass eine Auflösung für einen Vollschritt in Abhängigkeit einer Förderrate der Infusionspumpe angepasst wird.

Weiterhin können die Ansteuerungsdaten derart bestimmt werden, dass ein Maximalwert für einen Motorstrom des Schrittmotors vorgegeben wird.

Zudem können die Ansteuerungsdaten derart bestimmt werden, dass ein Motorstrom in Abhängigkeit einer Schrittgeschwindigkeit des Schrittmotors angepasst wird.

Zum Beispiel können die Ansteuerungsdaten derart bestimmt werden, dass der Motorstrom in Abhängigkeit von einem anliegenden Druck innerhalb der Infusionspumpe dynamisch angepasst wird.

Ferner können die Ansteuerungsdaten eine Vorgabe eines Haltestroms umfassen, der während einer Schrittpause auf den Schrittmotor wirken soll, sodass eine elektronische Rastposition während der Schrittpause aufrechterhalten wird.

Insbesondere können die Ansteuerungsdaten derart bestimmt werden, dass einzelne Mikroschritte eines Vollschritts kontinuierlich ausgegeben werden.

Dabei können die Ansteuerungsdaten derart bestimmt werden, dass bei Auftreten eines Drehmomentabrisses, ein Maximalwert für den Motorstrom für vorbestimmte Anzahl an Schritten erhöht wird und anschließend wieder erniedrigt wird, sodass ein erneuter Drehmomentabriss vermieden und eine weitere Geräuschentwicklung reduziert wird.

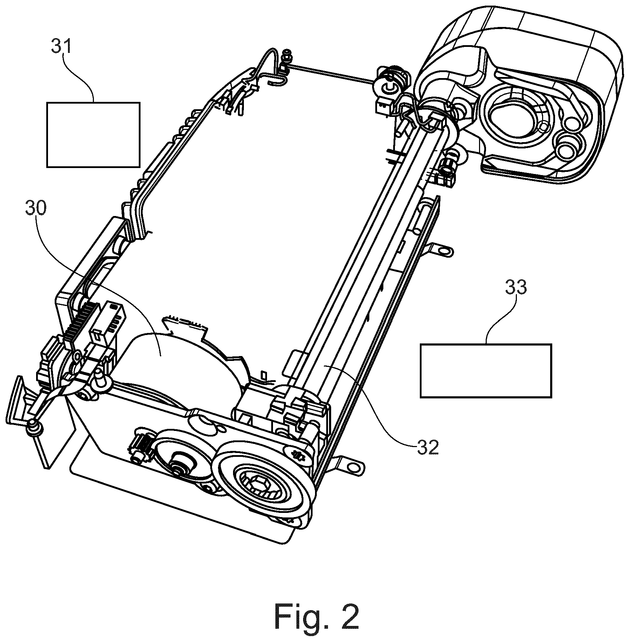

Figur 2 zeigt eine schematische Darstellung einer Infusionspumpe 33 mit einem Schrittmotor 30.

Der Schrittmotor 30 wird vorliegend von einer Ansteuerungsvorrichtung 31, die der Ansteuerungsvorrichtung 10 aus Fig. 1 entspricht, angesteuert. Die Ansteuerungsvorrichtung 31 ist vorliegend schematisch gezeichnet. Sie kann Teil der Motorsteuerung sein. Sie kann Hardware und Software umfassen. Sie kann eine Schnittstelle zum Datenaustausch mit dem Schrittmotor aufweisen. Der Schrittmotor 30 treibt über ein Getriebe eine Spindel innerhalb eines Antriebsarms 32 an. Der Antriebsarm 32 ist in Wirkverbindung mit einer Infusionspumpe 33. Die Infusionspumpe 33 ist eingerichtet eine medizinische Flüssigkeit (Bolus) auszugeben.

### Bezugszeichenliste

- 10, 31: Ansteuerungsvorrichtung
- 11: Empfangsbereich
- 12: Bestimmungsbereich
- 13: Ansteuerungsbereich

- 30: Schrittmotor
- 32: Antriebsarm
- 33: Infusionspumpe

## Patentansprüche

1. Ansteuerungsvorrichtung (10, 31) für einen Schrittmotor (30) einer Infusionspumpe (33), mit folgenden Vorrichtungs-Funktionsbereichen:
einem Empfangsbereich (11), der dafür vorgesehen und ausgebildet ist, Sollpositionsdaten für die Ansteuerung des Schrittmotors zu empfangen;
einem Bestimmungsbereich (12), der dafür vorgesehen und ausgebildet ist, Ansteuerungsdaten für den Schrittmotor auf Basis der Sollpositionsdaten zu bestimmen;
einem Ansteuerungsbereich (13), der dafür vorgesehen und ausgebildet ist, den Schrittmotor auf Basis der bestimmten Ansteuerungsdaten anzusteuern;
wobei der Bestimmungsbereich (12) vorgesehen und ausgebildet ist, die Ansteuerungsdaten derart zu bestimmen, sodass beim Betrieb der Infusionspumpe (33) eine Reduzierung der Geräuschbildung erzielt wird.

2. Ansteuerungsvorrichtung (10, 31) nach Anspruch 1,
wobei der Bestimmungsbereich (12) vorgesehen und ausgebildet ist, die Ansteuerungsdaten derart zu bestimmen, dass eine äquidistante Pulsweite vorliegt.

3. Ansteuerungsvorrichtung (10, 31) nach Anspruch 1 oder 2,
wobei der Bestimmungsbereich (12) vorgesehen und ausgebildet ist, die Ansteuerungsdaten derart zu bestimmen, dass eine Auflösung für einen Vollschritt in Abhängigkeit einer Förderrate der Infusionspumpe (33) angepasst wird.

4. Ansteuerungsvorrichtung (10, 31) nach einem der vorherigen Ansprüche,
wobei der Bestimmungsbereich (12) vorgesehen und ausgebildet ist, die Ansteuerungsdaten derart zu bestimmen, dass ein Maximalwert für einen Motorstrom des Schrittmotors vorgegeben wird.

5. Ansteuerungsvorrichtung (10, 31) nach einem der vorherigen Ansprüche,
wobei der Bestimmungsbereich (12) vorgesehen und ausgebildet ist, die Ansteuerungsdaten derart zu bestimmen, dass ein Motorstrom in Abhängigkeit einer Schrittgeschwindigkeit des Schrittmotors angepasst wird.

6. Ansteuerungsvorrichtung (10, 31) nach einem der vorherigen Ansprüche,
wobei der Bestimmungsbereich (12) vorgesehen und ausgebildet ist, die Ansteuerungsdaten derart zu bestimmen, dass der Motorstrom in Abhängigkeit von einem anliegenden Druck innerhalb der Infusionspumpe (33) dynamisch angepasst wird.

7. Ansteuerungsvorrichtung (10, 31) nach einem der vorherigen Ansprüche,
wobei der Bestimmungsbereich (12) vorgesehen und ausgebildet ist, die Ansteuerungsdaten derart zu bestimmen, dass sie eine Vorgabe eines Haltestroms umfassen, der während einer Schrittpause auf den Schrittmotor wirken soll, sodass eine elektronische Rastposition während der Schrittpause aufrechterhalten wird.

8. Ansteuerungsvorrichtung (10, 31) nach einem der vorherigen Ansprüche,
wobei der Bestimmungsbereich (12) vorgesehen und ausgebildet ist, die Ansteuerungsdaten derart zu bestimmen, dass einzelne Mikroschritte eines Vollschritts kontinuierlich ausgegeben werden.

9. Ansteuerungsvorrichtung (10, 31) nach einem der vorherigen Ansprüche,
wobei der Bestimmungsbereich (12) vorgesehen und ausgebildet ist, die Ansteuerungsdaten derart zu bestimmen, dass bei Auftreten eines Drehmomentabrisses, ein Maximalwert für den Motorstrom für eine vorbestimmte Anzahl an Schritten erhöht wird und anschließend wieder erniedrigt wird, sodass ein erneuter Drehmomentabriss vermieden und eine weitere Geräuschentwicklung reduziert wird.

10. Infusionspumpe (33) mit einem Schrittmotor (30) umfassend eine Ansteuerungsvorrichtung (10, 31) nach einem der Ansprüche 1 bis 9.

11. Ansteuerungsverfahren zur Ansteuerung eines Schrittmotors (30) einer Infusionspumpe (33), umfassend:
Empfangen von Sollpositionsdaten für die Ansteuerung des Schrittmotors (30);
Bestimmen von Ansteuerungsdaten für den Schrittmotor (30) auf Basis der Sollpositionsdaten;
Ansteuern des Schrittmotors (30) auf Basis der bestimmten Ansteuerungsdaten;
wobei die Ansteuerungsdaten derart bestimmt werden, sodass beim Betrieb der Infusionspumpe (33) eine Reduzierung der Geräuschbildung erzielt wird.

12. Computerprogramm, das Anweisungen aufweist, die bei Ausführung durch einen Computer diesen veranlassen, das Ansteuerungsverfahren gemäß Anspruch 11 auszuführen.

13. Computerlesbares Speichermedium, in dem das Computerprogramm gemäß Anspruch 12 gespeichert ist.
